(19) Europäisches Patentamt
European Patent Office
Office européen des brevets

(11) Veröffentlichungsnummer: **0 432 563 B1**

(12) **EUROPÄISCHE PATENTSCHRIFT**

(45) Veröffentlichungstag der Patentschrift: **09.03.94**

(51) Int. Cl.5: **C07D 277/50**, C07D 277/56, A01N 43/78, C07D 277/32

(21) Anmeldenummer: **90122585.4**

(22) Anmeldetag: **27.11.90**

(54) **4-Substituierte 5-Chlor-2-hydrazinothiazole.**

(30) Priorität: **09.12.89 DE 3940794**

(43) Veröffentlichungstag der Anmeldung:
**19.06.91 Patentblatt 91/25**

(45) Bekanntmachung des Hinweises auf die Patenterteilung:
**09.03.94 Patentblatt 94/10**

(84) Benannte Vertragsstaaten:
**BE CH DE FR GB IT LI NL**

(56) Entgegenhaltungen:
**EP-A- 0 348 735**
**FR-A- 2 039 748**

(73) Patentinhaber: **BAYER AG**

**D-51368 Leverkusen(DE)**

(72) Erfinder: **Beck, Gunter, Dr.**
**Am Mittelberg 19**
**W-5090 Leverkusen 1(DE)**
Erfinder: **Kysela, Ernst, Dr.**
**Virchowstrasse 14**
**W-5060 Bergisch-Gladbach 1(DE)**
Erfinder: **Braden, Rudolf, Dr.**
**Nordhauser Feld 1**
**W-5068 Odenthal(DE)**

EP 0 432 563 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 4-substituierte 5-Chlor-2-hydrazinothiazole und teilweise neue Zwischenprodukte zu ihrer Herstellung.

Es wurden neue, in 4-Stellung substituierte 5-Chlor-2-hydrazinothiazole der allgemeinen Formel (I)

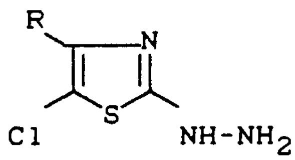

(I)

gefunden, in welcher

R für $CHF_2$, $CF_3$, CN oder $COOR^1$ steht,
wobei

$R^1$ für $C_1$-$C_4$-Alkyl steht.

Die Herstellung der neuen 5-Chlor-2-hydrazinothiazole (I) - nach an sich bekannten Verfahren - zeigt die folgende Formelübersicht:

**Formelübersicht:**

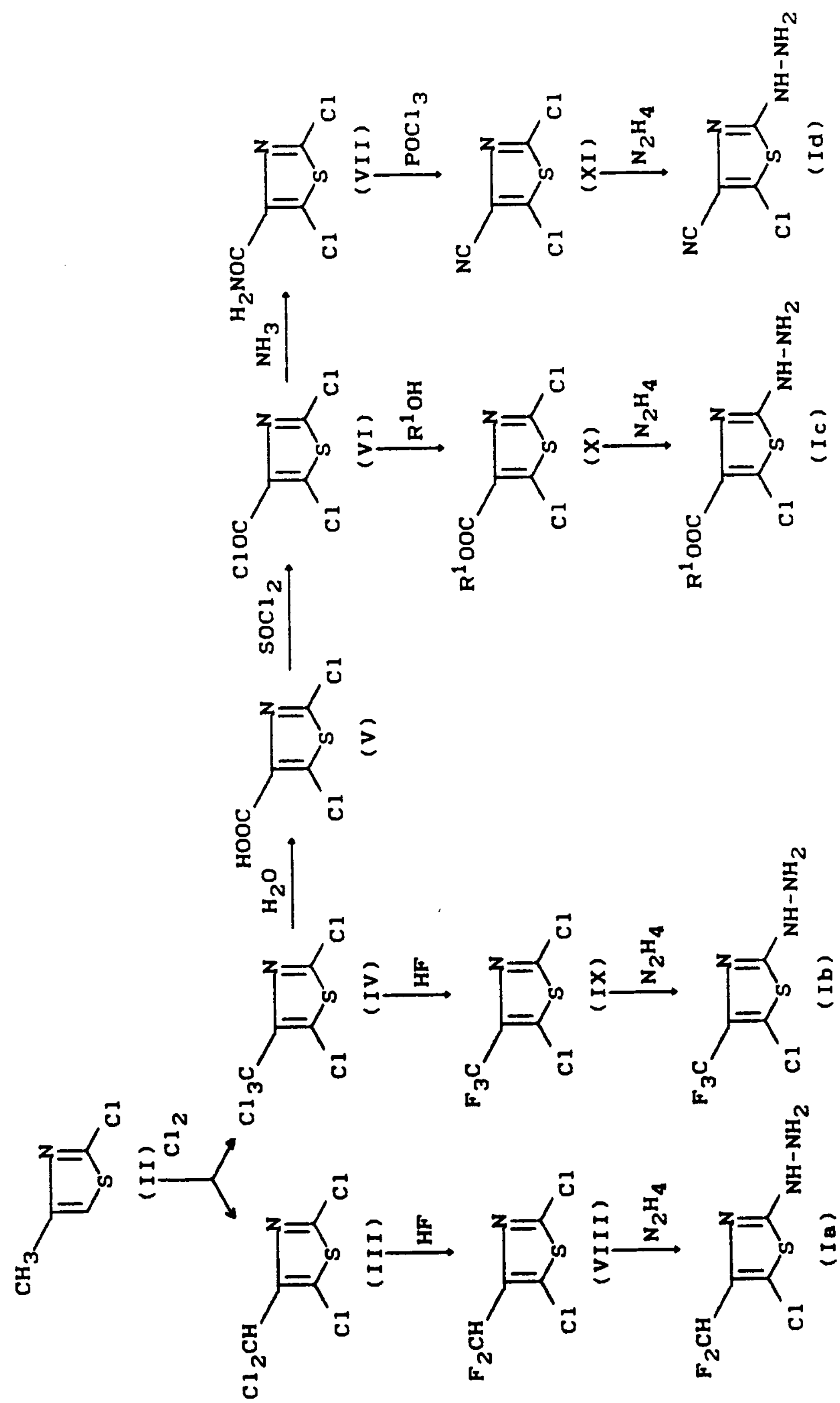


Ausgangsverbindung für alle 4-substituierten 5-Chlor-2-hydrazinothiazole (I) ist das bekannte 2-Chlor-4-methyl-thiazol der Formel (II) (vgl. J. Chem. Soc. 1919, S. 1071-1090).

Chlorierung von (II) bei einer Temperatur von etwa 100 °C führt überwiegend zum (noch nicht aus der Literatur bekannten) 2,5-Dichlor-4-dichlormethyl-thiazol der Formel (III), das ebenfalls Gegenstand der vorliegenden Erfindung ist.

Präparative Einzelheiten dieser Chlorierungsreaktion sowie aller anderen im Rahmen der Formelübersicht beschriebenen Reaktionen sind den nachfolgenden Beispielen zu entnehmen.

Chlorierung von (II) bis zu einer Endtemperatur von etwa 160 °C führt zum (noch nicht aus der Literatur bekannten) 2,5-Dichlor-4-trichlormethyl-thiazol der Formel (IV). Diese Verbindung ist Gegenstand einer eigenen älteren, nicht vorveröffentlichten DE-Patentanmeldung (P 38 21 598.5 vom 27.06.88/Le A 26 033).

Auch die aus (IV) erhältlichen Verbindungen der Formeln (V), (VI), (VII) und (XI) sind noch nicht aus der Literatur bekannt; sie sind ebenfalls Gegenstand der oben erwähnten eigenen älteren DE-Anmeldung P 38 21 598.5.

Die Carbonsäure der Formel (V) erhält man aus der Trichlormethylverbindung (IV) durch Umsetzung mit Wasser bei Temperaturen zwischen 80 °C und 100 °C.

Das Carbonsäurechlorid der Formel (VI) erhält man aus der Carbonsäure (V) durch Umsetzung mit einem Chlorierungsmittel, wie z.B. Thionylchlorid, bei Temperaturen zwischen 20 °C und 80 °C.

Das Carbonsäureamid der Formel (VII) erhält man aus dem Carbonsäurechlorid (VI) durch Umsetzung mit Ammoniak ($NH_3$) in Gegenwart eines inerten Verdünnungsmittels, wie z.B. Toluol, bei Temperaturen zwischen 0 °C und 40 °C.

Das Carbonsäurenitril der Formel (XI) erhält man aus dem Carbonsäureamid (VII) durch Umsetzung mit einem Dehydratisierungsmittel, wie z.B. Phosphoroxychlorid ($POCl_3$), bei Temperaturen zwischen 50 °C und 150 °C.

2,5-Dichlor-4-difluormethyl-thiazol der Formel (VIII) (vgl. die ältere, nicht vorveröffentlichte DE-Patentanmeldung P 38 21 600.0 vom 27.06.88/Le A 26 031 sowie EP-A-348 737) erhält man durch Umsetzung der Dichlormethylverbindung (III) mit wasserfreier Flußsäure (HF), gegebenenfalls unter Druck, bei Temperaturen zwischen 40 °C und 200 °C.

2,5-Dichlor-4-trifluormethyl-thiazol der Formel (IX) ist noch nicht aus der Literatur bekannt und ist auch Gegenstand der vorliegenden Erfindung. Seine Herstellung er folgt durch Umsetzung der Trichlormethylverbindung (IV) mit wasserfreier Flußsäure, gegebenenfalls unter Druck, bei Temperaturen zwischen 40 °C und 200 °C.

Die 2,5-Dichlor-4-thiazol-carbonsäureester der Formel (X) sind gleichfalls noch nicht aus der Literatur bekannt und sind ebenfalls Gegenstand der vorliegenden Erfindung. Ihre Herstellung erfolgt durch Umsetzung des Säurechlorids der Formel (VI) mit niederen ($C_1$-$C_4$) aliphatischen Alkoholen, zweckmäßig beim Siedepunkt des jeweiligen Alkohols.

Ein Alternativweg zur Herstellung der 2,5-Dichlor-4-thiazolcarbonsäureester der Formel (X) besteht in der Umsetzung der Trichlormethylverbindung (IV) mit dem betreffenden niederen aliphatischen Alkohol in der Siedehitze und in Gegenwart katalytischer Mengen (ca. 1 Gew.-%) von wasserfreiem Eisen(III)-chlorid.

Die Herstellung der neuen, erfindungsgemäßen 4-substituierten 5-Chlor-2-hydrazinothiazole (Ia) bis (Id) erfolgt in an sich bekannter Weise durch Umsetzung der entsprechenden 2-Chlorthiazole (VIII), (IX), (X) und (XI) (vgl. Formelübersicht) mit Hydrazin(hydrat) in einem geeigneten organischen Lösungsmittel, wie z.B. $C_1$-$C_3$-Alkohole oder (cyclische) Ether wie Dioxan, bei Temperaturen zwischen 0 °C und 40 °C.

Die erfindungsgemäßen 4-substituierten 5-Chlor-2-hydrazinothiazole der Formel (I) besitzen eine fungizide Wirksamkeit, insbesondere gegen Pyricularia oryzae am Reis, Venturia inaequalis (z.B. an Obst), Phytophthora infestans (z.B. an Tomaten), Plasmopara viticola an Reben sowie Pellicularia sasakii am Reis.

Darüber hinaus können die Thiazole (I) auch als Zwischenprodukte zur Herstellung von bestimmten Azofarbstoffen eingesetzt werden, erhältlich durch oxidative Kupplung mit entsprechenden Anilinen. Die resultierenden 4-Aminophenyl-azothiazole können zum Färben und Bedrucken von Fasermaterialien sowie für den Sublimationstransferdruck verwendet werden (vgl. DE-A-38 04 814).

Die als Zwischenprodukte benötigten, neuen 4-substituierten 2,5-Dichlorthiazole der Formeln (III), (IX) und (X) können auch gemeinsam definiert werden durch die Formel (XII):

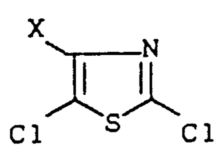

(XII),

in welcher

X für $CHCl_2$, $CF_3$ oder $COOR^1$ steht, wobei

$R^1$ für $C_1$-$C_4$-Alkyl steht.

Die Zwischenprodukte der Formel (XII) sind ebenfalls Gegenstand der Erfindung.

Die Verbindungen der Formel (XII) können außerdem auch als Zwischenprodukte zur Herstellung von herbizid wirksamen Thiazolyloxyacetamiden verwendet werden (vgl. z.B. EP-A-18 497, US-A-4 645 525, EP-A-195 237, US-A-4 788 291).

Darüber hinaus besitzen die Verbindungen der Formeln (IX) und (X) ebenfalls eine fungizide Wirksamkeit, insbesondere gegen Plasmopara viticola, Phytophthora infestans, Pyricularia oryzae und Venturia inaequalis.

Die nachfolgenden Beispiele dienen zur weiteren Erläuterung der Erfindung.

Herstellungsbeispiele

Beispiel 1

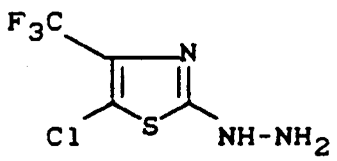

(Ib)

Eine Mischung aus 111 g (0,5 Mol) 2,5-Dichlor-4-trifluormethyl-thiazol (IX) und 500 ml Dioxan wurde unter Rühren und unter leichter Kühlung in dem Maße mit 75 g (1,5 Mol) Hydrazinhydrat versetzt, daß eine Reaktionstemperatur von 25 ° C nicht überschritten wurde. Nach 20-stündigem Nachrühren bei Raumtemperatur wurde das Reaktionsgemisch in 2,5 l Eiswasser eingerührt, dann wurde abfiltriert, der Filterrückstand mit Wasser gewaschen und getrocknet.

Man erhielt 87,3 g (80,3 % der Theorie) 5-Chlor-2-hydrazino-4-trifluormethyl-thiazol vom Schmelzpunkt 136-137 ° C.

Beispiel 2

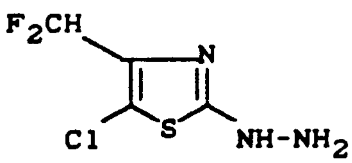

(Ia)

Analog zum 5-Chlor-2-hydrazino-4-trifluormethyl-thiazol (Ib) wurde aus 2,5-Dichlor-4-difluormethyl-thiazol (VIII) in 51,3 %iger Ausbeute das 5-Chlor-4-difluormethyl-2-hydrazinothiazol (Ia) erhalten, Schmelzpunkt 132 ° C (Zers.) (nach Umkristallisieren aus viel Cyclohexan).

Kleinere Mengen der Verbindung (Ia) lassen sich schon bei 70 ° C/0,1 mbar sublimieren.

Beispiel 3

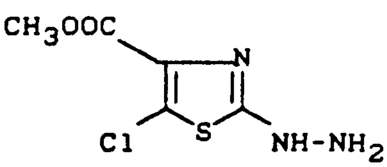

(Ic, $R^1$ = $CH_3$)

Eine Mischung aus 30,3 g (0,143 Mol) 2,5-Dichlor-4-thiazolcarbonsäuremethylester (X, $R^1$ = $CH_3$) und 150 g Dioxan wurde mit 14,5 g (0,29 Mol) Hydrazinhydrat versetzt und das Reaktionsgemisch wurde drei Stunden bei Raumtemperatur kräftig gerührt. Anschließend wurde in 1,5 l Wasser verrührt, abfiltriert, mit Wasser gewaschen und getrocknet. Ausbeute 6,5 g (21,9 % der Theorie) 5-Chlor-2-hydrazino-4-thiazolcarbonsäuremethylester vom Schmelzpunkt 196,5 ° C (Zers.).

Beispiel 4

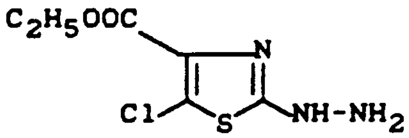

(Ic, $R^1$ = $C_2H_5$)

Eine Mischung aus 40,0 g (0,177 Mol) 2,5-Dichlor-4-thiazolcarbonsäureethylester (X, $R^1$ = $C_2H_5$) und 175 g Dioxan wurde bei Raumtemperatur unter Rühren mit 26,5 g (0,53 Mol) Hydrazinhydrat versetzt. Nach 20-stündigem Nachrühren bei Raumtemperatur wurde in 1250 ml Eiswasser verrührt, abfiltriert, mit Wasser gewaschen und getrocknet. Ausbeute 13,9 g (35,5 % der Theorie) 5-Chlor-2-hydrazino-4-thiazolcarbonsäureethylester vom Schmelzpunkt 190 ° C (Zers.).

Beispiel 5

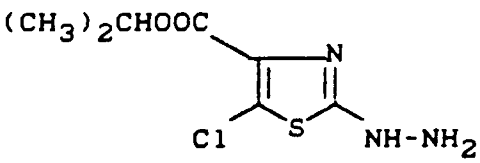

(Ic, $R^1$ = i-$C_3H_7$)

Eine Lösung von 24,0 g (0,1 Mol) 2,5-Dichlor-4-thiazolcarbonsäure-isopropylester (X, $R^1$ = i-$C_3H_7$) in 100 g Dioxan wurde unter Rühren mit 10,0 g (0,2 Mol) Hydrazinhydrat versetzt, Nach 15-stündigem Nachrühren bei Raumtemperatur wurde in ca. 500 ml Wasser verrührt, abfiltriert, mit Wasser gewaschen und getrocknet. Ausbeute 11,8 g (50,1 % der Theorie) 5-Chlor-2-hydrazino-4-thiazolcarbonsäure-isopropylester vom Schmelzpunkt 156-157 ° C (Zers.).

Beispiel 6

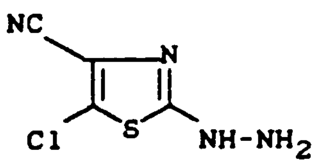

(Id)

Eine Lösung von 17,9 g (0,1 Mol) 4-Cyano-2,5-dichlor-thiazol (XI) in 100 ml Methanol wurde mit einer Mischung aus 10 g (0,2 Mol) Hydrazinhydrat und 50 ml Methanol versetzt. Nach etwa 5 Minuten begann sich aus der leicht erwärmten Reaktionslösung ein Niederschlag abzuscheiden. Nach Rühren über Nacht wurde in ca. 750 ml Wasser verrührt, abfiltriert, mit Wasser gewaschen und getrocknet. Ausbeute 15,2 g (87,1 % der Theorie) 5-Chlor-4-cyano-2-hydrazino-thiazol;Schmelzpunkt 225 ° C (Zers.) (umkristallisiert aus Toluol).

Beispiel 7

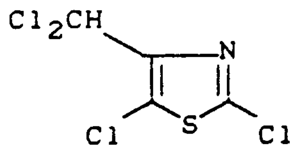

(III)

In 5888 g (42,9 Mol) 97,3 %iges 2-Chlor-4-methyl-thiazol (II) wurde anfangs bei 80 °C, nach Abklingen der exothermen Reaktion dann bei 100 °C etwa 100 Stunden lang ein kräftiger Chlorstrom geleitet. Nach dem Abkühlen auf Raumtemperatur und Stehen über Nacht entstand ein kristalliner Niederschlag von 2,5-Dichlor-4-dichlormethyl-thiazol (III), der abfiltriert und auf Ton getrocknet wurde. Ausbeute 3907 g (38,4 % der Theorie).

Der Flüssiganteil des Chlorierungsgemischs wurde an einer 2m-Füllkörperkolonne fraktioniert destilliert. Das bei 101 °C bis 103 °C/6 mbar erhaltene Destillat kristallisierte weitgehend aus und wurde anschließend auf Ton getrocknet. Ausbeute an 2,5-Dichlor-4-dichlormethyl-thiazol (III) durch fraktionierte Destillation: 2733 g (26,9 % der Theorie).

Gesamtausbeute 6640 g (65,3 % der Theorie). Schmelzpunkt 42 °C-44 °C (umkristallisiert aus wenig Petrolether).

$^1$H-NMR (in $CDCl_3$): $\delta$ = 6,78 ppm.

Beispiel 8

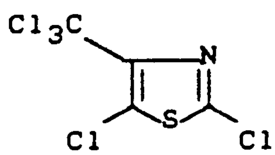

(IV)

In einem Dreihalskolben, der mit Rührer, Thermometer, Rückflußkühler und Gaseinleitungsrohr vorsehen war, wurde, beginnend bei Raumtemperatur, in eine Mischung aus 1093 g (8,19 Mol) 2-Chlor-4-methyl-thiazol und 4 l Methylenchlorid Chlorgas eingeleitet. Nach Abklingen der exothermen Reaktion wurde unter allmählicher Temperatursteigerung und weiterem Einleiten von Chlor zunächst das Methylenchlorid abdestilliert und dann der Sumpf langsam bis auf etwa 160 °C erhitzt. Bei etwa 160 °C wurde dann so lange meist überschüssiges Chlorgas (erkennbar an der leicht grünlichen Farbe des Abgases) eingeleitet, bis im Gaschromatogramm fast nur noch die gewünschte Ver bindung 2,5-Dichlor-4-trichlormethyl-thiazol zu erkennen war. Gesamtdauer der Chlorierung 40 bis 50 Stunden.

Eine Grobdestillation bis zu einer Kopftemperatur von 150 °C bei 14 mbar erbrachte 2057 g ca. 95 %iges 2,5-Dichlor-4-trichlormethyl-thiazol, was einer Ausbeute von 88 % der Theorie, bezogen auf reines Produkt, entspricht. Das 2,5-Dichlor-4-trichlormethyl-thiazol wurde durch eine Feindestillation an einer ca. 220 cm langen, silberverspiegelten Füllkörperkolonne rein erhalten. Siedepunkt 123 °C-125 °C bei 16 mbar.

Beispiel 9

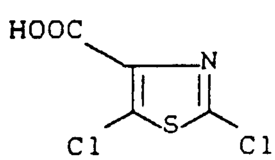

(V)

271,5 g (1 Mol) 2,5-Dichlor-4-trichlormethyl-thiazol und 2700 ml Wasser wurden unter Rühren über Nacht (etwa 15 Stunden) unter Rückfluß erhitzt. Nach dem Abkühlen auf Raumtemperatur wurde der entstandene

kristalline Niederschlag abfiltriert, mit Wasser gewaschen und getrocknet.

Ausbeute 143,5 g (72,5 % der Theorie) 2,5-Dichlor-thiazol-4-carbonsäure. Die Verbindung ist bei 120 °C/0,1 mbar sublimierbar und z.B. aus Chloroform umkristallisierbar. Schmelzpunkt 191 °C unter Zersetzung.

Durch Einengen der wäßrigen Phase konnten weitere 30,5 g etwas weniger reines Produkt isoliert werden.

Beispiel 10

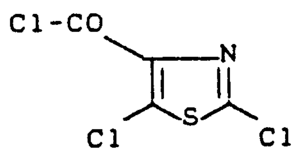

(VI)

Eine Mischung aus 143,5 g (0,725 Mol) 2,5-Dichlor-thiazol-4-carbonsäure und 700 ml Thionylchlorid wurde unter Rühren langsam erhitzt. Bereits bei etwa 40 °C trat starke Gasentwicklung ein. Im Verlauf einer halben Stunde wurde bis auf Rückflußtemperatur erhitzt und dort bis zum Ende der Gasentwicklung gehalten (ca. 2 Stunden): Endtemperatur etwa 80 °C.

Nach dem Abziehen des überschüssigen Thionylchlorids im Wasserstrahlvakuum hinterblieben 144,6 g (92,2 % der Theorie) kristallines 4-Chlorcarbonyl-2,5-dichlor-thiazol. Aus Petrolether große, farblose Kristalle vom Schmelzpunkt 58 °C - 59 °C.

Beispiel 11

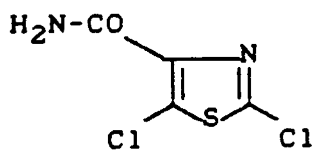

(VII)

Über eine Lösung von 144,6 g (0,668 Mol) 4-Chlorcarbonyl-2,5-dichlor-thiazol (VI) in 750 ml wasserfreiem Toluol wurden unter Rühren und Kühlung bei maximal 20 °C 61 g (3,59 Mol) Ammoniakgas geleitet. Anschließend wurde der entstandene Niederschlag abfiltriert, mit Toluol gewaschen, getrocknet und etwa eine Stunde in 1 l Eiswasser verrührt

Nach dem Abfiltrieren, Waschen mit Wasser und Trocknen wurden 127,9 g (97,2 % der Theorie) 2,5-Dichlor-thiazol-4-carboxamid vom Schmelzpunkt 153 °C erhalten.

Beispiel 12

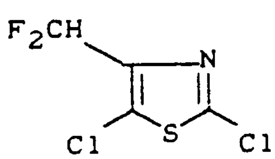

(VIII)

1010 g (4,26 Mol) 2,5-Dichlor-4-dichlormethyl-thiazol (III) wurden mit 1500 ml wasserfreier Flußsäure im VA-Autoklaven bei 145 °C/25 bar fluoriert. Der entstehende Chlorwasserstoff wurde laufend entspannt. Nach Ende der Reaktion wurde die überschüssige Flußsäure im Vakuum bei Raumtemperatur abgezogen. Der Rückstand wurde auf Eiswasser gegeben, in Dichlormethan aufgenommen, über Natriumsulfat getrocknet und destilliert.

Man erhielt 753 g (86,6 % der Theorie) 2,5-Dichlor-4-difluormethyl-thiazol (VIII), Siedepunkt 74-75 °C/18 mbar; $n_D^{20}$ = 1,5171.

Beispiel 13

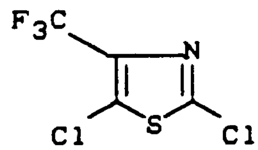

(IX)

750 9 (2,76 Mol) 2,5-Dichlor-4-trichlormethyl-thiazol (IV) wurden mit 1000 ml wasserfreier Flußsäure im VA-Autoklaven bei 130 °C/19-20 bar fluoriert. Der entstehende Chlorwasserstoff wurde laufend entspannt. Nach Ende der Reaktion wurde die überschüssige Flußsäure im Vakuum bei Raumtemperatur abgezogen. Der Rückstand wurde auf Eiswasser gegeben, in Dichlormethan aufgenommen, über Natriumsulfat getrocknet und destilliert.

Man erhielt 570 g (93 % der Theorie) 2,5-Dichlor-4-trifluormethyl-thiazol; Siedepunkt: 164 °C; $n_D^{20}$ = 1,4774.

Beispiel 14

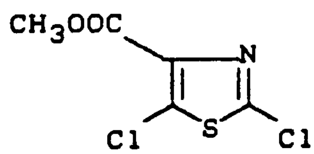

(X, $R^1$ = $CH_3$)

Eine Lösung von 100 g 4-Chlorcarbonyl-2,5-dichlor-thiazol (VI) in 500 ml Methanol wurde 15 Minuten zum Sieden erhitzt. Nach Abziehen des überschüssigen Methanols bei ca. 25 °C im Vakuum hinterblieben 97 9 (99 % der Theorie) farbloser, öliger 2,5-Dichlor-4-thiazolcarbonsäure-methylester, der beim Stehen zu einer weißen Kristallmasse vom Schmelzpunkt 46 °C erstarrte.

Beispiel 15

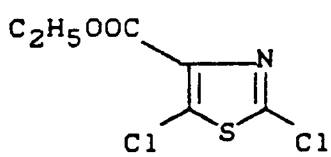

(X, $R^1$ = $C_2H_5$)

In analoger Weise wie bei Beispiel 14 wurde 2,5-Dichlor-4-thiazolcarbonsäure-ethylester als farbloses Öl erhalten.

Die acht intensivsten IR-Banden (in $cm^{-1}$): 1725, 1498, 1453, 1325, 1307, 1206, 1056 und 1020.

Beispiel 16

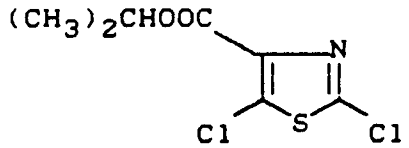

(X, $R^1$ = i-$C_3H_7$)

In analoger Weise wie bei Beispiel 14 wurde 2,5-Dichlor-4-thiazolcarbonsäure-isopropylester vom Schmelzpunkt 46 ° C erhalten.

Beispiel 17

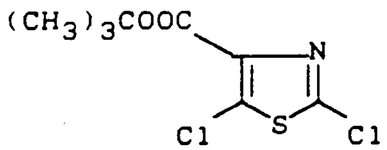

(X, $R^1$ = t-$C_4H_9$)

In analoger Weise wie bei Beispiel 14 wurde 2,5-Dichlor-4-thiazolcarbonsäure-tertiärbutylester vom Schmelzpunkt 190 ° C-191 ° C (Zers.) erhalten.

Beispiel 18

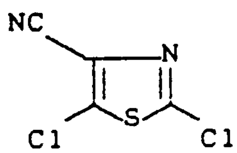

(XI)

127,8 g (0,649 Mol) 2,5-Dichlor-thiazol-4-carboxamid (VII) und 1300 ml Phosphoroxychlorid wurden unter Rühren etwa 5 Stunden unter Rückfluß (ca. 110 ° C) erhitzt. Nach dem Abdestillieren der Hauptmenge überschüssigen Phosphoroxychlorids im Wasserstrahlvakuum wurde der ölige Rückstand tropfenweise zu etwa 1 l Wasser gegeben, das durch Kühlung zwischen 15 ° C und 20 ° C gehalten wurde. Der dabei entstehende kristalline Niederschlag wurde abfiltriert, mit Wasser gewaschen und getrocknet.

Man erhielt so 103 g (88,7 % der Theorie) 4-Cyano-2,5-dichlorthiazol. Die Verbindung ist bei 114 ° C/16 mbar destillierbar, bei 80 ° C/16 mbar sublimierbar und schmilzt bei 56,5 ° C-57 ° C.

Verwendungsbeispiele

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Wirkstoffe, Konzentrationen und Testergebnisse gehen aus der nachfolgenden Tabelle A hervor.

Tabelle A

Pyricularia-Test (Reis) / protektiv

| Wirkstoff (Beispiel Nr.) | Wirkungsgrad in % der unbehandelten Kontrolle bei einer Wirkstoffkonzentration von 250 ppm |
|---|---|
| 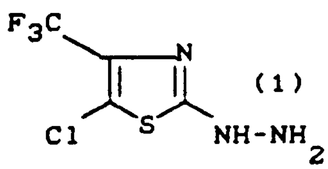 (1) | 100 |
| 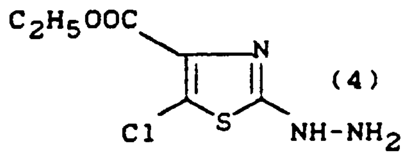 (4) | 89 |
| 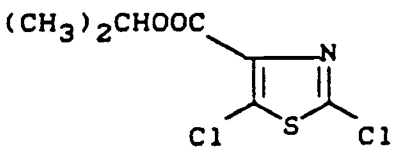 (16) | 80 |
| 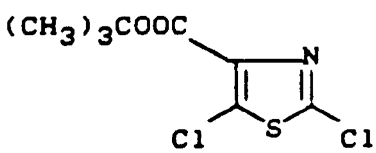 (17) | 90 |

Beispiel B

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Wirkstoffe, Konzentationen und Testergebnisse gehen aus der nachfolgenden Tabelle B hervor.

Tabelle B

Venturia-Test (Apfel) / protektiv

| Wirkstoff (Beispiel Nr.) | Wirkungsgrad in % der unbehandelten Kontrolle bei einer Wirkstoffkonzentration von 250 ppm |
|---|---|
| 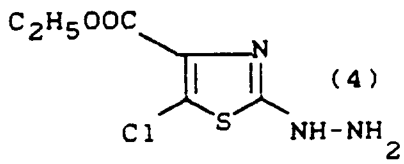 (4) | 67 |
| 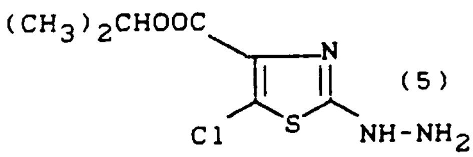 (5) | 80 |
| 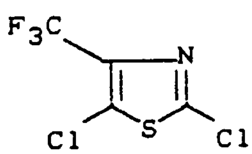 (13) | 100 |
| 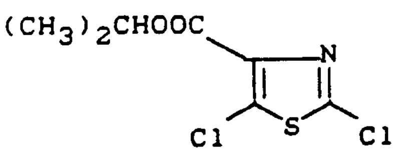 (16) | 70 |
| 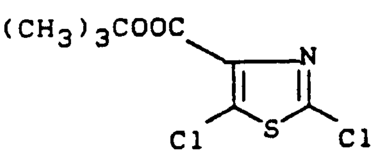 (17) | 50 |

Beispiel C

Phytophthora-Test (Tomate) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Wirkstoffe, Konzentrationen und Testergebnisse gehen aus der nachfolgenden Tabelle C hervor.

Tabelle C

Phytophthora-Test (Tomate) / protektiv

| Wirkstoff (Beispiel Nr.) | Wirkungsgrad in % der unbehandelten Kontrolle bei einer Wirkstoffkonzentration von 250 ppm |
|---|---|
| 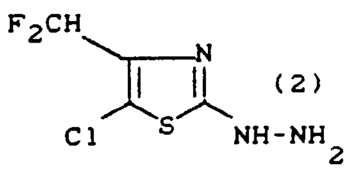 (2) | 50 |
| 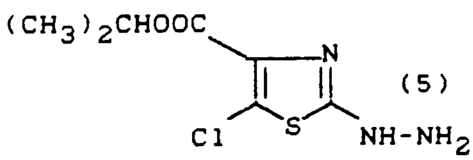 (5) | 67 |
| 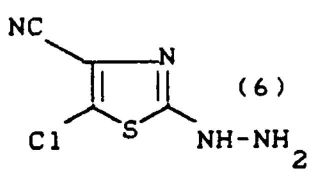 (6) | 72 |
| 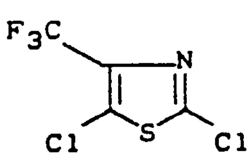 (13) | 67 |

Beispiel D

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Wirkstoffe, Konzentrationen und Testergebnisse gehen aus der nachfolgenden Tabelle D hervor.

Tabelle D

Plasmopara-Test (Reben) / protektiv

| Wirkstoff (Beispiel Nr.) | Wirkungsgrad in % der unbehandelten Kontrolle bei einer Wirkstoffkonzentration von 10 ppm |
|---|---|
| 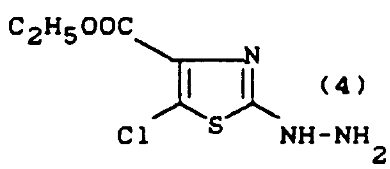 (4) | 60 |
| 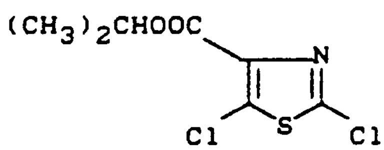 (16) | 90 |

Beispiel E

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

Wirkstoff, Konzentrationen und Testergebnisse gehen aus der nachfolgenden Tabelle E hervor.

Tabelle E

Pellicularia-Test (Reis)

| Wirkstoff (Beispiel Nr.) | Wirkstoff-konzentration | Wirkungsgrad in % der unbehandelten Kontrolle |
|---|---|---|
| 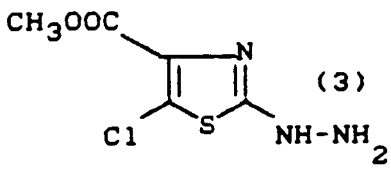 (3) | 250 ppm<br>50 ppm | 80<br>70 |

## Patentansprüche

**1.** 4-Substituierte 5-Chlor-2-hydrazinothiazole der Formel (I),

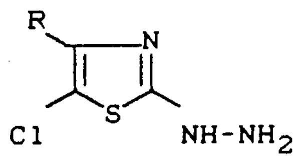

(I)

in welcher
R für $CHF_2$, $CF_3$, CN oder $COOR^1$ steht, wobei
$R^1$ für $C_1$-$C_4$-Alkyl steht.

**2.** 4-Substituierte 2,5-Dichlorthiazole der Formel (XII),

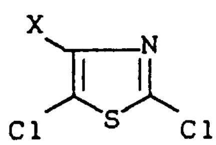

(XII),

in welcher
X für $CHCl_2$, $CF_3$ oder $COOR^1$ steht, wobei
$R^1$ für $C_1$-$C_4$-Alkyl steht.

## Claims

**1.** 4-Substituted 5-chloro-2-hydrazinothiazoles of the formula (I)

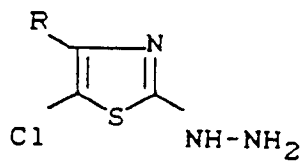

(I)

in which

R represents $CHF_2$, $CF_3$, CN or $COOR^1$,
where
$R^1$ represents $C_1$-$C_4$-alkyl.

**2.** 4-Substituted 2,5-dichlorothiazoles of the formula (XII)

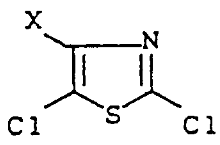

(XII),

in which

X represents $CHCl_2$, $CF_3$ or $COOR^1$,
where
$R^1$ represents $C_1$-$C_4$-alkyl.

## Revendications

**1.** 5-Chloro-2-hydrazinothiazoles substitués en position 4, de formule I

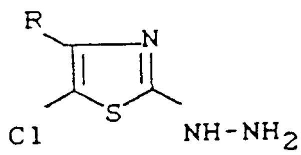

(I)

dans laquelle

R représente $CHF_2$, $CF_3$, CN ou $COOR^1$,
$R^1$ représentant un groupe alkyle en $C_1$-$C_4$.

**2.** 2,5-Dichlorothiazoles substitués en position 4, de formule XII

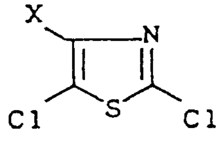

(XII),

dans laquelle

X représente $CHCl_2$, $CF_3$ ou $COOR^1$,
$R^1$ représentant un groupe alkyle en $C_1$-$C_4$.